Europäisches Patentamt

European Patent Office

Office européen des brevets

(19)

(11) Publication number: **0 194 789**
**A2**

## (12) EUROPEAN PATENT APPLICATION

(21) Application number: 86301463.5

(22) Date of filing: 28.02.86

(51) Int. Cl.⁴: **G 01 N 33/543**
**C 12 Q 1/00, G 01 N 33/535**
**//G01N33/569, G01N33/571**

(30) Priority: 28.02.85 US 706826
14.11.85 US 798347

(43) Date of publication of application:
17.09.86 Bulletin 86/38

(84) Designated Contracting States:
BE CH DE FR GB IT LI NL SE

(71) Applicant: Becton Dickinson and Company
Mack Centre Drive P.O. Box 2224
Paramus New Jersey 07652-1149(US)

(72) Inventor: Jachimowicz, Joanna D.

deceased(US)

(72) Inventor: McFarland, Edward C.
1388 Deanwood Road
Baltimore Maryland(US)

(72) Inventor: Lovell, Stephen J.
724 A-7 Camberly Circle
Towson Maryland(US)

(72) Inventor: Mulligan, James J., Jr.
409 Tanglewood Court
Joppa Maryland(US)

(74) Representative: Ruffles, Graham Keith et al,
MARKS & CLERK 57-60 Lincoln's Inn Fields
London WC2A 3LS(GB)

(54) Apparatus and method for multiple simultaneous assay.

(57) A diagnostic system and a method for the simultaneous assay of a plurality of samples to detect a ligand is provided. The diagnostic system includes a plurality of reaction chambers which can be aligned. A dipstick card containing a plurality of dipsticks is provided. The dipsticks are in a configuration which permits simultaneous insertion of the dipsticks into an aligned set of chambers. The diagnostic system is particularly suitable for immunoassay and nucleic acid hybridization techniques.

FIG.1.

EP 0 194 789 A2

# APPARATUS AND METHOD FOR MULTIPLE SIMULTANEOUS ASSAY

The present application is a continuation-in-part of United States Patent Application Serial Number 706,826, filed on February 28, 1985.

## BACKGROUND OF THE INVENTION

### 1. Field of the Invention

The present invention relates generally to diagnostic apparatus in which a fluid sample is to be tested. More particularly, the present invention is directed to apparatus and a method for the simultaneous analysis of a plurality of fluid samples suspected of containing a ligand, such as a component of an antibody-antigen reaction.

The method and apparatus of the present invention provides a relatively simple, inexpensive and reliable means for testing a plurality of individual body fluid samples of patients for a variety of ligands, such as those patients suspected of having contracted an infectious disease, such as M. tuberculosis, C. difficile, campylobacter, rotavirus, Chlamydia or N. gonorrhoeae.

The method and apparatus permit testing a plurality of body fluid samples simultaneously with a simple, easily understood dipstick format using prepackaged reagents in a configuration that does not require precise measurements or proportions.

## 2. Prior Art

The present method and apparatus is particularly suitable for detection of antigens and antibodies by the enzyme linked immunoabsorbent assay (ELISA) method described in United States Patent Numbers 3,654,090, 3,791,932, 3,850,752, 3,839,153, 3,879,262 and 4,016,043 of Schuurs et al. The method and apparatus are also suitable for other immunoassay procedures and other assay procedures, such as nucleic acid hybridization techniques.

The use of individual dipsticks in immunoassay procedures is well known. An example of such an immunoassay dipstick procedure is a commercially available pregnancy test from Quidel, Inc., Santa Monica, California. The dipstick of this pregnancy test is a plastic strip with two pads attached to one end. One pad is an indicator pad having antibody against beta hCG attached thereto. The other pad is an integral negative control. In use, the dipstick is immersed in a first tube into which a fluid sample has been placed and which contains a dissolved reagent. The dipstick is removed from the first tube, is rinsed with water and is immersed in a second tube having an indicator reagent. The indicator pad turns blue compared to the control pad if the result is positive.

While such dipstick immunoassay procedures can provide several advantages, such as ease of administration and quickness of result; the currently available dipstick procedures have the disadvantage that they require individual, discrete manipulation of each patient sample.

Among the objects of the present invention are to provide a novel method of performing simultaneous assays of body fluid specimens, such as serum, urine or the like, to detect the presence of reactive components; to provide such a method utilizing inexpensive materials and which can be carried out with more ease and a more simple manner than in previous assay methods; to provide a test kit for use in performing simultaneous assays which is readily produced in quantity and which will reduce the time involved in performing a plurality of assays and to provide such a test kit which contains the required reagents for the assay in predispensed form.

## SUMMARY OF THE INVENTION

The present invention includes a diagnostic system for the simultaneous assay of a plurality of samples. The diagnostic system can be packaged in a format permitting the simultaneous assay for one or for a plurality of reactive components.

In general, the diagnostic system includes a plurality of reaction vessels. Each reaction vessel has a plurality of separate chambers for receiving the various reagents required for an assay. The separate chambers are generally disposed in a linear array proceeding from a first chamber to a second chamber and then to an $n^{th}$ chamber in consecutive numbered order. For most applications, the total number of chambers need not be more than six. The reaction vessels have a configuration such that the first chamber and each higher numbered chamber can be placed in alignment.

The diagnostic system also includes a plurality of dipsticks. The dipsticks are joined in a configuration which permits simultaneous insertion of the dipsticks into the first chamber and each higher numbered chamber as desired when the chambers are aligned.

Optionally the diagnostic system may include means for aligning the chambers in a configuration suitable for receiving insertion of the joined dipsticks.

## DESCRIPTION OF THE DRAWINGS

FIG. 1 is a perspective schematic view of the components of one embodiment of the present invention showing the components prior to assembly into an operating relationship; and

FIG. 2 is an enlarged perspective view of one embodiment of a series of chambers of the diagnostic system of the invention in a format suitable for an enzyme immunoassay.

FIG. 3 is a perspective view of another embodiment of the present invention showing the components prior to assembly into an operating relationship; and

FIG. 4 is a perspective view of a further embodiment of the present invention showing the components prior to assembly into an operating relationship.

## DETAILED DESCRIPTION OF THE INVENTION

It should be understood that the apparatus of the invention can be used for a variety of assays, such as immunochemical and nucleic acid hybridization assays. For convenience, however, the following detailed description will sometimes be illustrated by use of the apparatus with an immunochemical technique.

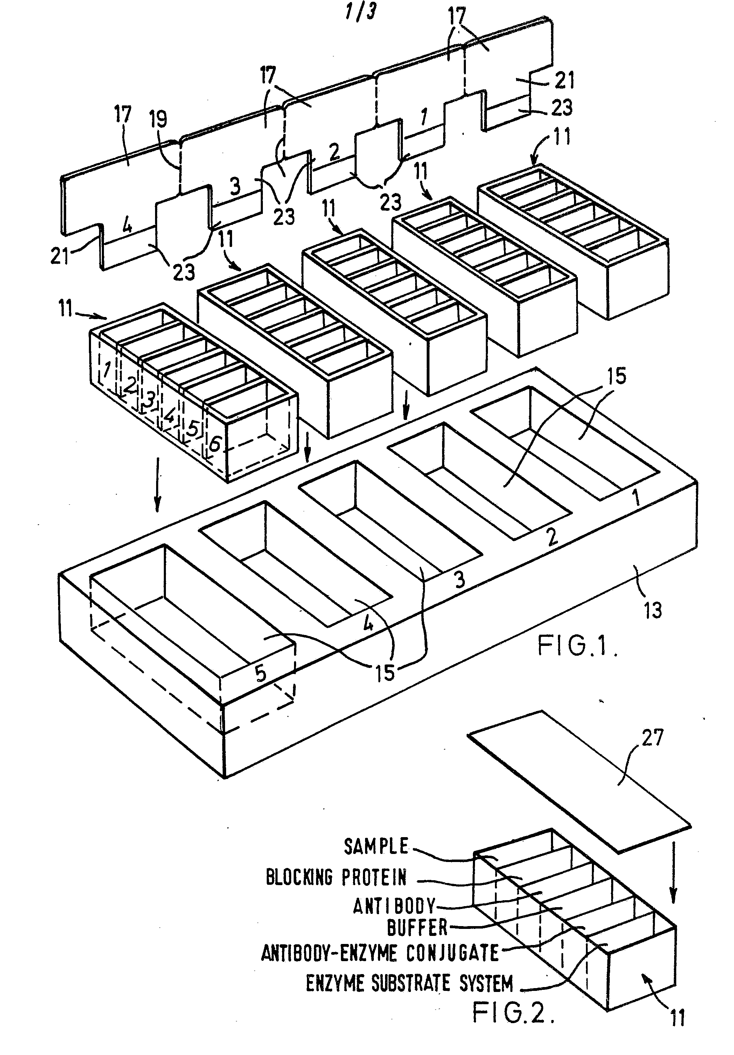

A plurality of reaction vessels 11 are shown in FIG. 1. In the embodiment shown in Figure 1, each reaction vessel has 6 chambers. For ease of explanation, one of the reaction vessels shows the chambers labeled from 1 to 6, although such labelling is not required for use of the diagnostic system.

The reaction vessel has a configuration such that the chambers of a plurality of reaction vessels can be placed in alignment. As shown in

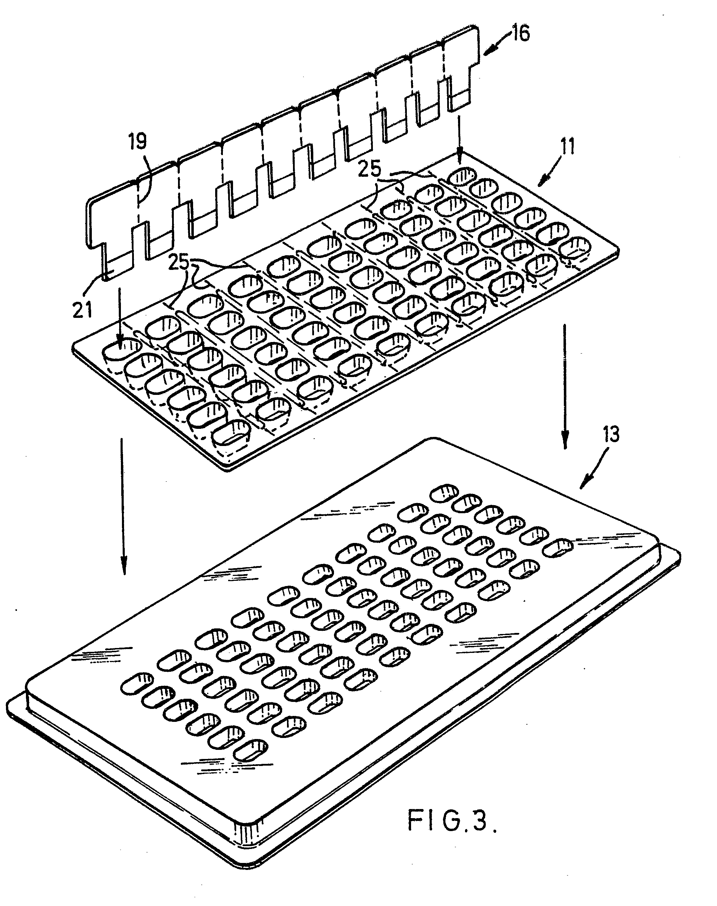

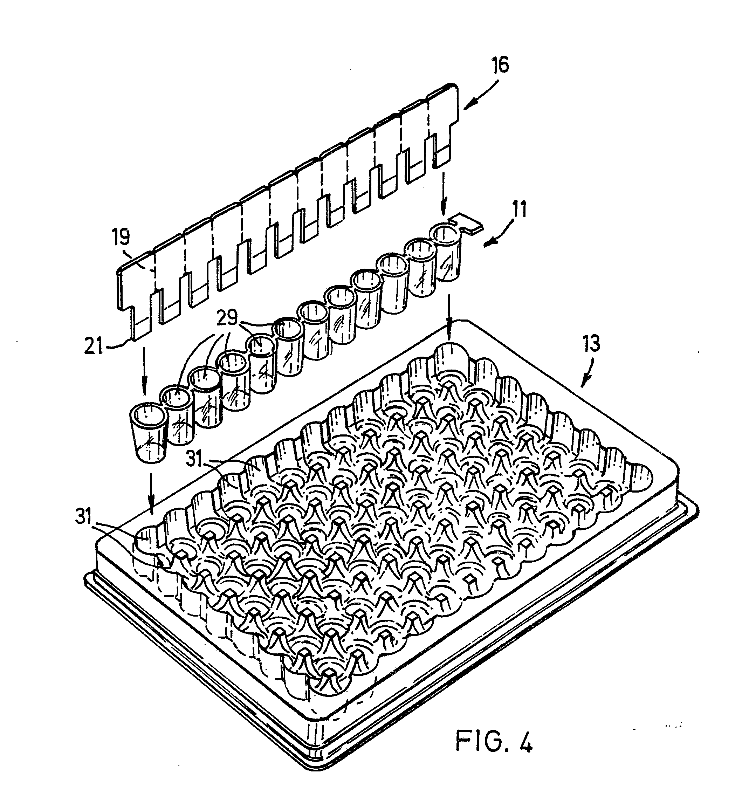

FIG. 1, the reaction vessels have a rectangular parallelepiped construction and are aligned in a rectangular, planar array with the first and each higher numbered chamber being in linear alignment. However, curvilinear and non-planar alignment formats are equally suitable, if such alignments are considered desirable for aesthetic or marketing reasons. Other means for effecting alignment are shown in FIGS. 3 and 4.

As shown in FIG. 1, a tray 13 is provided as the means for aligning the reaction vessels 11. The tray 13 is provided with a plurality of recesses 15 for receiving the reaction vessels 11. The recesses 15 are located in the tray so as to effect a suitable alignment of the reaction vessels 11 when they are placed in the recesses. As shown in FIG. 1, the recesses 15 are preferably marked with a numerical indicia to aid in keeping track of the results of an assay performed with a particular reaction vessel.

The tray 13 may be of a size suitable for handling any desired number of reaction vessels. For practical handling reasons the tray 13 is of a size suitable for forming from about 5 to about 20 recesses therein. In use, any one or all of the recesses may be loaded with a reaction vessel. Since it is usually desirable to run a positive and a negative control with each assay run, the minimum number of recesses loaded with reaction vessels is usually three.

A dipstick card 16 containing a plurality of joined dipsticks 17 is provided for insertion into the compartments of the reaction vessels 11. As shown in FIG. 1 the dipsticks are formed from a single piece of a suitable material, preferably a suitable plastic, such as polystyrene or polyvinyl chloride. The plastic is scored (dotted lines 19) to permit removing those dipsticks which are not required. The number of dipsticks formed corresponds to the number of recesses provided in tray 13.

As shown in FIG. 1. the dipstick 17 includes an elongated finger 21 which is shaped and spaced in a configuration suitable for insertion into an aligned set of compartments of the reaction vessels 11. The tips of the elongated fingers 21 are covered or coated with a suitable material 23 capable of binding an immunoreactive component. It has been found that nitrated cellulose paper is a particular suitable material for this purpose. Polymeric materials, such as described in U.S. Patent No. 4,450,231 to Onkau and Eeva-Marjatha Saloner et al., "Immobilization of Viral and Mycoplasen Antigens And Immunoglobulins On Polystyrene Surface For Immunoassays, Journal of Immunological Methods 30, (1979) 209-218 have also been found to be suitable. It should be understood that the covering on the tips 21 does not have to be selective for the immunoreactive component of interest so long as it is capable of binding a suitable proportion of the immunoreactive component.

The diagnostic system of the invention is preferably packaged to provide a single tray, a plurality of reaction vessels and a number of dipstick cards which is suitable for the utilization of the reaction vessels in an immunoassay. In a preferred embodiment, as shown in FIG. 3, the diagnostic assembly is a package which includes a tray with 60 recesses, 10 reaction vessels, each reaction vessel having 6 chambers and a dipstick card having 10 individual joined dipsticks. More than one set of reaction vessels and a corresponding number of dipstick cards may be provided with each package. As shown in FIG. 3 the 10 reaction vessels are joined in an array. An individual set of reaction vessels may, if desired, be separated from the array by cutting or breaking the score line 25.

A further embodiment of the invention is shown in FIG. 4. As shown in FIG. 4, the tray 13 has a series of wells 31 which are adapted to receive individual chambers 29. When the individual reaction vessels are placed into the wells 31, a plurality of aligned chambers is provided.

In most cases, the diagnostic system will include a negative control and a positive control in a bulk amount. The controls are added to two of the chambers in lieu of a sample to provide a negative and positive color for precise identification of immunoassay results.

The diagnostic system of the present invention is particularly adapted for use with the ELISA immunoassays previously mentioned. The most general form of an ELISA test consists of incubating the serum or other sample to be tested with a solid phase base to permit the binding of an immunoreactive component in the specimen to the solid phase. An enzyme labeled antibody which recognizes the immunoreactive component is added and permitted to incubate for a desired period of time, followed by washing with a suitable solution. A substrate is then added which will react with the enzyme now attached to the solid phase and normally produce a color change, dependent on the amount of immunoreactant which has been bound. The amount and rate of color change is related to the amount of immunoreactant which is present in the sample. The color change may be observed visually for a generally qualitative determination. If desired, the color change may be observed with a suitable instrument, such as a fluorometer or spectrophotometer to provide a more accurate reading which can be used to provide both a qualitative determination and a quantitative determination, when suitable standards are developed.

The diagnostic system of the present invention may also be used with a liposome color readout, such as is described in pending United States Patent Application Serial No. 579,667, filed February 14, 1984, which is commonly assigned to the assignee of the present invention and which is incorporated herein by reference. For use in the present invention, a liposome is used to encapsulate a quantity of dye. The liposome is conjugated to a ligand, such as an antibody, and is used to replace the enzyme-antibody conjugate previously described. Further details with respect to the preparation of liposomes are set forth in U.S. Patent No. 4,342,846, which is incorporated herein by reference. Suitable dye labels are described in U.S. Patent No. 4,373,932, which is incorporated herein by reference.

In the method of the invention, the diagnostic system is used for the simultaneous immunoassay of a plurality of samples. After the chambers of the reaction vessel are aligned, a plurality of fluid body samples are placed in a first aligned set of chambers. The dipstick card is manipulated so as to insert the elongated fingers of a plurality of dipsticks into each of the set of chambers containing a sample. The dipsticks are incubated in the sample containing chambers for a period sufficient to cause binding of a ligand to the coating on the dipstick. As used herein, for immunochemical detection, the term ligand means an immunoreactive analyte selected from the group consisting of antibody, antigen or hapten. In the case of nucleic acid detection, the term ligand refers to a strand of nucleic acid containing a complementary sequence of nucleotides.

In some immunoassays, the ligand is an antigen which can bind directly to a solid support, such as nitocellulose. Since subsequent reagents are used which are specific for the bound antigen, it is immaterial whether other components of the sample become bound to the coating. Since the detecting reagents may bind to unreacted sites on the coating, an intermediate blocking step is used. In the blocking step, the dipsticks with bound antigen are incubated in the presence of a nonreactive protein, such as bovine serum albumin, which binds to the unreacted sites and prevents the unreacted sites from entering into subsequent steps.

In other cases, an anti-ligand immunoreactive component, such as an antibody, which is specific for the ligand is first bound to the coating on the dipstick and the unreacted sites on the coating are blocked with a nonreactive protein prior to packaging the dipsticks. After the ligand is bound to the dipstick, subsequent steps are directed to binding an anti-ligand immunoreactive component which is linked to a detecting substance, such as an enzyme or liposome, to the ligand.

In some cases, it is preferable to use an indirect method to detect bound antigen. The first detecting antibody (A) is specific for antigen. Binding of antibody (A) to antigen is monitored by the second detecting antibody (B) which is specific for antibody (A). Antibody (B) is conjugated with the detecting substance which may be an enzyme, fluorophore, liposome, etc.

When an enzyme is used as the detecting substance, a substrate is provided with which the enzyme reacts in a separate developing step to provide a color. When the enzyme selected is a phosphatase, the substrate can be p-nitrophenyl phosphate in which case the solution becomes colored, or a mixture of 5-bromo-4-chloro-3-indolyl phosphate and nitro blue tetrazolium in which case the colored product is deposited on the solid support. If the enzyme selected is a peroxidase, the substrate can be ortho-phenylene-diamine with a small amount of hydrogen peroxide (for colored solution), or 4-chloro-1-naphthol/hydrogen peroxide (for colored solid support). When the enzyme selected is B-galactosidase, the substrate can be a galactoside; for other types of antibody coupled enzymes, an appropriate substrate should, of course, be utilized. The selection of a suitable substrate is within the knowledge of one skilled in the art of immunoassay procedures.

Some of the various stable reagents required for an immunoassay in accordance with the invention may be predispensed into the reaction vessel compartments prior to packaging the reaction vessels. The top of the reaction vessels are then covered with a removable cover 27 until the reaction vessel is used.

A typical series of reagents used in the practice of the present invention is shown in FIG. 2.

M&C FOLIO: 799P51626                    WANGDOC: 0657C

CLAIMS:

1. A diagnostic system for the simultaneous assay of a plurality of samples to detect a ligand, the diagnostic system comprising:

    (a)   a plurality of chambers adapted to receive assay reagents, the chambers being generally disposed in a configuration proceeding from a first chamber to a second chamber and consecutively to an $n^{th}$ chamber, and the configuration of the chambers being such that the first chamber, the second chamber and the succeeding chambers can be placed in alignment; and

    (b)   a dipstick card containing a plurality of dipsticks joined in a configuration permitting sumultaneous insertion of the dipsticks into the first chambers, the second chambers and the succeeding chambers as desired when the chambers are aligned.

2. A diagnostic system in accordance with claim 1 which further includes means for aligning the chambers in a configuration adapted to receive the dipsticks, the aligning means preferably being a tray having a plurality of recesses for receiving the chambers in a configuration which aligns the chambers.

3. A diagnostic system in accordance with claim 1 or 2 wherein a plurality of chambers are joined into an array of pre-aligned chambers.

4. A diagnostic system in accordance with claim 1, 2 or 3 wherein the dipstick card permits removal of dipsticks which are not required for an assay.

5. A diagnostic system in accordance with any preceding claim, wherein each of the dipsticks terminates in an elongate finger which is capable of binding a ligand.

6. A diagnostic system in accordance with claim 5 wherein the elongate fingers are covered on the terminal end with a material capable of binding a ligand, preferably cellulose nitrate paper or cellulose acetate paper.

7. A diagnostic system in accordance with claim 5 or 6 wherein an anti-ligand immunoreactive component has been bound to the elongate fingers.

8. A diagnostic system in accordance with claim 7 wherein unreacted binding sites on the elongate fingers are blocked with a non-reactive protein.

9. A diagnostic method for the simultaneous immunoassay of a plurality of samples to detect a ligand, the method comprising

(a)  providing a plurality of chambers, the chambers being generally disposed in a configuration proceeding from a first chamber to a second chamber and then consecutively to an $n^{th}$ chamber and the configuration of the chambers being such that the first chamber, the second chamber and each higher chamber can be placed in alignment;

(b)  providing a dipstick card containing a plurality of dipsticks joined in a configuration permitting simultaneous insertion of the dipsticks in consecutive order into the first chambers, the second chambers and higher chambers as desired when the chambers are aligned;

(c)  aligning the chambers in a configuration adapted to receive the joined dipsticks;

(d)   placing a liquid sample in each of a first aligned set of chambers;

(e)   inserting the joined dipsticks into the first aligned set of chambers containing the samples and incubating the dipsticks in the chambers in a manner to allow binding of ligand to the dipsticks;

(f)   placing an anti-ligand reactive component capable of being bound to the ligand in a second aligned set of chambers, the anti-ligand component being linked to a detecting substance capable of producing a visually detectable colour;

(g)   inserting the joined dipsticks into the second aligned set of chambers and incubating the dipsticks in the chambers in a manner to cause binding of the detecting substance to the ligand; and

(h)   simultaneously determining the presence of absence of the ligand in each of the liquid samples by the presence or absence of th visually detectable colour.

10. A method in accordance with claim 9 wherein the detecting substance is an enzyme, preferably horseradish peroxidase, alkaline phosphatase or β-glactosidase.

11. A method in accordance with claim 9 wherein the detecting subtance is a lipsome.

12. A method in accordance with claim 9, 10 or 11 wherein an anti-ligand reactive component is placed in a second aligned set of chambers, an anti-anti-ligand immunoreactive component is placed in a third aligned set of chambers, the anti-anti-ligand component being linked to a detecting substance capable of producing a visually detectable colour, and the joined dipsticks are consecutively incubated in the second and the third set of chambers to allow binding of the detecting substance to the ligand.

13. A method in accordance with any of claims 9 to 12, wherein the assay is an immunossay or a nucleic acid hybridization assay.

0194789

1/3

FIG.1.

SAMPLE
BLOCKING PROTEIN
ANTIBODY
BUFFER
ANTIBODY-ENZYME CONJUGATE
ENZYME SUBSTRATE SYSTEM

FIG.2.

FIG.3.

FIG. 4